# EUROPEAN PATENT APPLICATION

(11) **EP 2 990 486 A1**
(43) Date of publication of application: **02.03.2016**
(21) Application number: 15182570.0
(22) Date of filing: 26.08.2015
(51) Int. Cl.: C12N 15/09, A01H 1/04, A01H 5/00

(54) **DOUBLE FLOWER TRAIT AND COLD-PERIOD INDEPENDENT, MOPHEAD HYDRANGEA**

(30) Priority: 29.08.2014 US 201414473514
(71) Applicant: Hydrangea Breeders Association BV, 1424 PR De Kwakel (NL)
(72) Inventor: ARTS, Niels, 1424 PR De Kwakel (NL)
(74) Representative: von Kreisler Selting Werner - Partnerschaft von Patentanwälten und Rechtsanwälten mbB

(57) **Abstract**

The instant disclosure provides Hydrangea plants and methodology for producing Hydrangea plants having any combination of the traits double flower, mophead inflorescence, and cold-period independent flowering. Illustrative Hydrangea plants include (1) cold-period independent flowering with mophead inflorescence; (2) cold-period independent flowering with double flowers; and (3) cold-period independent flowering with mophead inflorescence and with double flowers.

## Description

### RELEVANT FIELD

The present disclosure relates to breeding new, distinct, and stable traits into Hydrangea cultivars. Provided herein are plants and methodology for introducing any combination of double flowering, cold-period independent flowering, and mophead inflorescence into Hydrangea.

### INTRODUCTION

Hydrangea macrophylla belongs to the family of the Hydrangeaceae. Hydrangea macrophylla cultivars are popular garden plants and are also used to produce flowering potted plants and cut flowers. The biggest commercial use is as potted plants. Cultivars with new and stable traits are developed through controlled breeding programs, leading to more attractive flower and inflorescence traits, longer flowering periods, more sturdy stems and reduced production costs, among others.

Hydrangea inflorescences come in various forms. The classic form is the "Teller" (German for disc) or "lacecap", which is a flat, large inflorescence consisting of a rim with a relatively small number of big-tepaloid flowers and a large centre of relatively many flowers with very small sepals and petals. Other forms are known as well, such as the plume- or coneshaped inflorescence, which is characteristic for Hydrangea paniculata and consists only of tepaloid flowers. The third form is the mophead inflorescence, which has the shape of a half or full sphere and also contains only big tepaloid flowers. The mophead inflorescence is highly desired for the production of flowering potted plants and cut flowers, because they are showier than the other types of inflorescences.

Hydrangea flowers come in two basic forms: small flowers with petals and anthers that easily drop and large flowers with 4-5 tepaloid sepales, 4-5 petals and 5-10 anthers that also easily drop. The large tepaloid flowers are the most attractive ones for commercial production. Normally, each tepaloid flower has only 4-6 under- or fully-developed tepaloids, but cultivars have been bred, e.g. Hydrangea macrophylla "Corsage", "Wedding Gown" and "Komachi", in which other flower parts like the usually small petals and stamens are also modified to small or large tepaloids, giving the flowers up to any number between 6 and 20 small to large tepaloids. This trait is called double flower.

### SUMMARY

In one aspect, the present disclosure provides a Hydrangea plant having one or more traits selected from the group consisting of cold-period independent flowering, mophead inflorescence, and double flower. In one embodiment, the plant has the traits cold-period independent flowering and double flower. In another embodiment, the plant has the traits cold-period independent flowering and mophead inflorescence. In another embodiment, the plant has the traits cold-period independent flowering, mophead inflorescence, and double flower.

In another aspect, provided is methodology for breeding a Hydrangea hybrid plant having the traits cold-period independent flowering, mophead inflorescence, and double flower comprising: (a) crossing a cold-period independent flowering Hydrangea macrophylla plant, either as male or female parent, with any Hydrangea macrophylla plant; and (b) selecting a plant from the progeny of such cross that flowers within 8 months of potting the rooted cuttings due to the presence of the cold-period independent flowering trait and exhibits the traits mophead inflorescence and double flower.

In another aspect, provided is methodology for breeding a Hydrangea hybrid plant having the traits cold-period independent flowering, and double flower, comprising: (a) crossing a cold-period independent flowering Hydrangea macrophylla plant, either as male or female parent, with any Hydrangea macrophylla plant; and (b) selecting a plant from the progeny of such cross that flowers within 8 months of potting the rooted cutting due to the presence of the cold-period independent flowering trait and exhibits the trait double flower.

### BRIEF DESCRIPTION OF THE DRAWINGS

**FIGURE 1****:** Typical lacecap inflorescence showing a more or less flat arrangement of relatively few big tepaloid single flowers at the rim of the inflorescence and relatively many non-showy flowers at the centre of the inflorescence.
**FIGURE 2****:** Typical half spherical mophead inflorescence showing only big tepaloid single flowers.
**FIGURE 3****:** Detail of a tepaloid single flower from a lacecap inflorescence showing 5 tepaloids, 3 petals, 6 stamens and 2 stigmas.
**FIGURE 4****:** Detail of a non-showy central flower from a lacecap inflorescence showing 6 petals, 10 stamens and 3 stigmas.
**FIGURE 5****:** Detail of a single flower showing 5 almost equally sized tepaloids, 4 petals, 7 anthers and 2 stigmas.
**FIGURE 6****:** Detail of a double flower with a low degree of doubleness, showing 8 almost equally sized tepaloids and 4 stigmas.
**FIGURE 7****:** Detail of a double flower with a high degree of doubleness, showing 19 big to very small tepaloids and 4 stigmas.
**FIGURE 8****:** Lacecap inflorescence with the double flower trait with a high degree of doubleness in rim and central flowers.
**FIGURE 9****:** Spherical mophead inflorescence with double flowers and a high degree of doubleness.
**FIGURE 10****:** Tepaloids from a double flower with a low degree of doubleness. The most outer tepaloid is the upper left one, the most inner tepaloid is the bottom right one.
**FIGURE 11****:** Tepaloids from a double flower with a high degree of doubleness. The most outer tepaloid is the upper left one, the most inner tepaloid is the bottom right one.

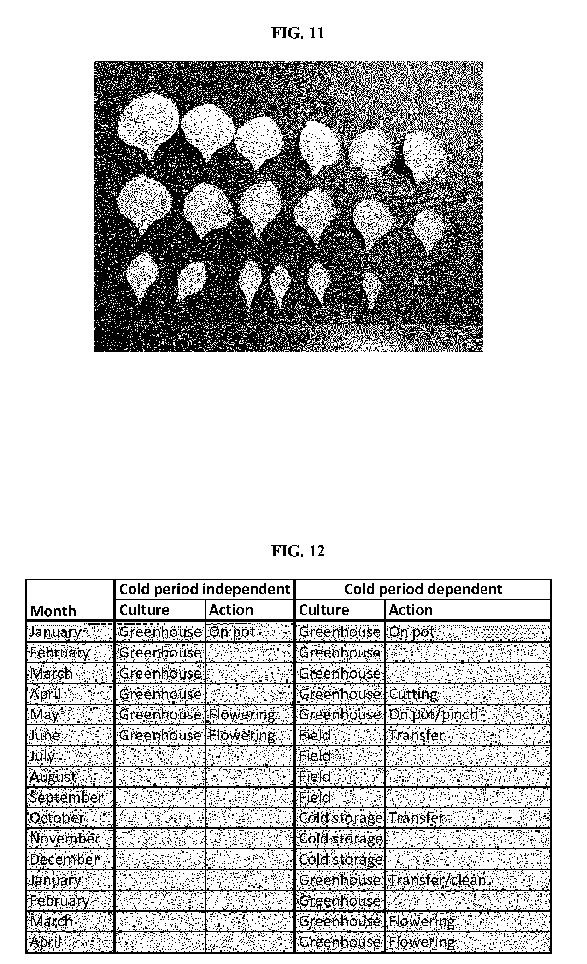

**FIGURE 12****:** Scheme for selection on cold-period independent flowering and other flowering traits in Hydrangea macrophylla progenies in North-West Europe. For other regions the scheme may deviate. The scheme starts with potting rooted cuttings from the seedlings in January and culturing those under greenhouse conditions. Selections that flowered in May, June stayed in the greenhouse. The non-flowering ones were transferred to the field for further culture, cold treated at 2-5 °C in the months October to December, and returned to the greenhouse in January. Flowering was observed in March and April.

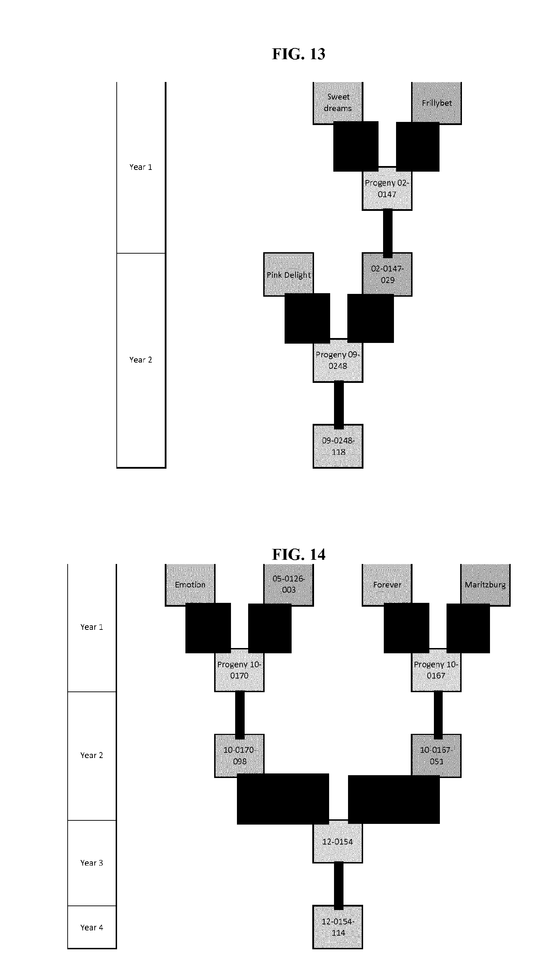

**FIGURE 13****:** Breeding scheme for producing Hydrangea having cold-independent flowering, mop-head inflorescence, and rigid stems. Red is female parent, blue is male parent, grey is progeny, green is selected new potential cultivar.
**FIGURE 14****:** Breeding scheme for producing Hydrangea having recessive trait double flower with rigid stems, compact growth, and small leaves. Red is female parent, blue is male parent, grey is progeny, green is selected new potential cultivar.

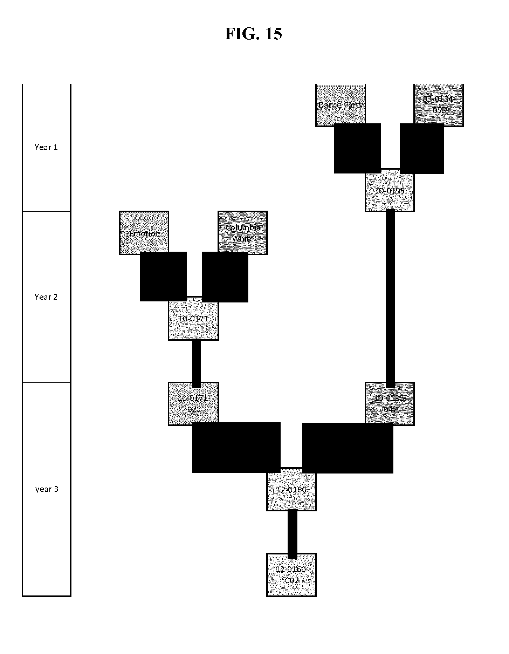

**FIGURE 15****:** Breeding scheme for producing Hydrangea having recessive traits double flower and mophead, along with cold-period independent flowering, rigid stems, compact growth, and small leaves in a short period of time. Red is female parent, blue is male parent, grey is progeny, green is selected new potential cultivar.

### DETAILED DESCRIPTION

Mass production of new Hydrangea cultivars is achieved by vegetative propagation using cuttings. Cuttings are taken from well-selected mother plants and subsequently rooted in trays under relatively high humidity. The rooted cuttings are planted in pots and grown in the field or the greenhouse. The pot size determines the size of the flowering potted plant at the end of the culture. The potted plants are pinched once or twice to induce branching. On these branches flower buds will develop under short-day conditions, but these buds will go into dormancy. Therefore, at a certain stage of development, the potted plants are moved into a cooling house at 2-5 °C for 6-8 weeks to break the dormancy of the flower buds. After this cold storage, the plants are moved to the greenhouse, where they are grown under long-day conditions to flowering stage. The cold treatment also synchronizes the flowering of all the branches.

This production scheme has several disadvantages. From planting the cuttings in the final pot size to reaching the fully flowering stage needed for sale takes 9-13 months, depending on location and season. It also requires a lot of handling; moving potted plants from the greenhouse or field to the cold storage room and back again. Furthermore, the cold storage itself may negatively affect the quality of the plant caused by fungal attack or by the well-known phenomenon of bud rot.

Thus far, traditional breeding has failed to produce Hydrangea macrophylla cultivars that stably express the trait combination of cold-period independent flowering with the mophead inflorescence containing only double flowers. The major reasons probably are the dominancy of the lacecap inflorescence over the mophead inflorescence, the recessive inheritance of the double-flower trait, the quantitative character of the cold-period-independent-flowering trait, and the load of unwanted traits coming from parents carrying these interesting traits, e.g. weak and bending stems, very big leaves, long period between potting and reaching full flowering stage.

The present disclosure provides Hydrangea plants that have any combination of the traits double flower, mophead inflorescence, and cold-period independent flowering. The instant disclosure also provides methodology to produce new Hydrangea plants with any combination of the traits double flower, mophead inflorescence, and cold-period independent flowering by crossing one plant exhibiting one, two, or all of these traits with another plant containing none, one, two, or all of these traits, and selecting progeny that exhibits one of the following combinations: (1) cold-period independent flowering with mophead inflorescence; (2) cold-period independent flowering with double flowers; and (3) cold-period independent flowering with mophead inflorescence and with double flowers.

In so doing, and as discussed below, Applicant provides Hydrangea plants and methodology for stably and reliably producing Hydrangea plants that have any combination of the traits double flower, mophead inflorescence, and cold-period independent flowering. For example, and in one embodiment, the instant disclosure provides a Hydrangea plant that exhibits the traits cold-period independent flowering and either mophead inflorescence or double flower. In another embodiment, an illustrative Hydrangea plant exhibits the traits cold-period independent flowering, mophead inflorescence, and double flower.

The instant disclosure also provides methodology for producing a Hydrangea plant having cold-period independent flowering and one or both of the traits mophead inflorescence and double flower and at least one second desirable trait, the method comprising crossing, as male or female parent, a Hydrangea plant having one, two or all of the traits cold-period independent flowering, mophead inflorescence and double flower, with a second Hydrangea plant having at least one other desirable trait, and selecting progeny having at least one of the traits cold-period independent flowering, mophead inflorescence and double flower in combination with the other desirable trait. This other desirable trait may be stem rigidness, stem branching, disease resistance/tolerance, inflorescence size, and flower colour, among others.

The combination cold-period independent flowering with at least one of double flowers and mophead inflorescence is of very high commercial value. Using the instant plants and methodology, potted flowering plants and cut-flowers of cultivars carrying any combination of the cold-period independent flowering trait with double flowers and mophead inflorescence can be produced faster and against strongly reduced costs.

Technical terminology in this description conforms to common usage in botany and plant taxonomy.

As used herein, **cold-period independent flowering** is defined as the ability of a plant to produce a fully flowering inflorescence within 8 months after potting the rooted cutting and without the need to interrupt the 15 °C or higher growing conditions with a cold period at 2-8 °C. Many Hydrangea cultivars develop under long-day conditions buds in which the meristem develops into an inflorescence. The further development of the inflorescence stops as the buds go dormant. To break this dormancy, plants are moved to a cold room for a minimum of 6 weeks and then returned to the normal growing conditions. After this cold period, the inflorescence meristems will develop further into fully flowering inflorescences. In Hydrangea plants that express the cold-period independent flowering trait, the buds containing the inflorescence meristems do not go dormant at the normal growing conditions and, subsequently, do not need a cold-period to further develop the inflorescence meristems into full flowering inflorescences. Cold-period independent flowering means that the flower buds on the plant do not need a cold period to grow into a fully flowering inflorescence.

Cold-period independent flowering reduces the culture time needed to produce a flowering potted plant and a cut flower by more than half and reduces the substantial labour needed to transport the plants to and from the cold storage.

As stated herein, cold-period independent flowering refers to the ability to flower within 8 months after potting the rooted cuttings. Of course, and as understood by one of ordinary skill in the art, within 8 months inherently encompasses flowering within about 3 months, 4 months, 5 months, 6 months, 6 months, 7 months, and 8 months. Additionally, and as clear from the instant disclosure and Examples, the flowers are produced on first year wood.

As used herein, a **tepaloid** is a showy flower part that looks like a petal and is positioned in the flower on the place where normally a bracteole, sepal, petal, or anther is situated, and which may have various colours, which may include green, different sizes, the biggest ones normally situated towards the outside of the flower and the smallest ones normally situated towards the centre of the flower, and various forms, which may include ovate, obovate, lanceolate, rhombic, kidney-shaped and fimbriate, amongst others.

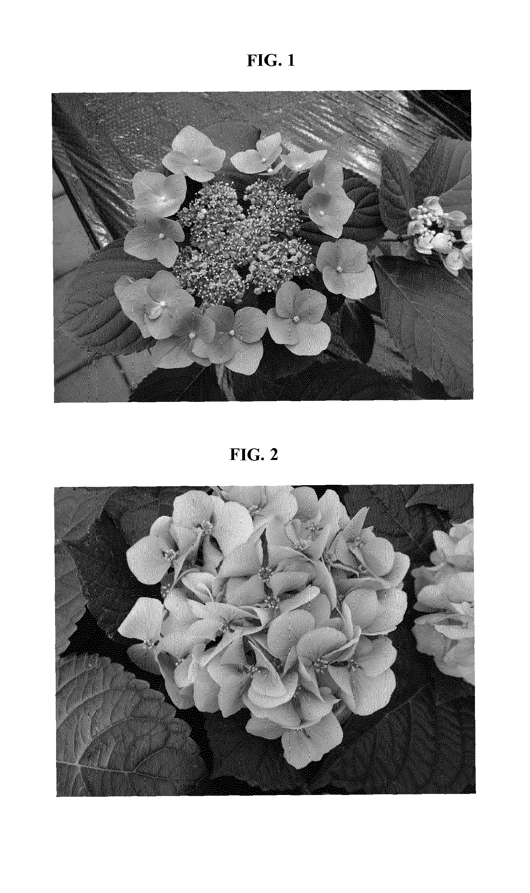

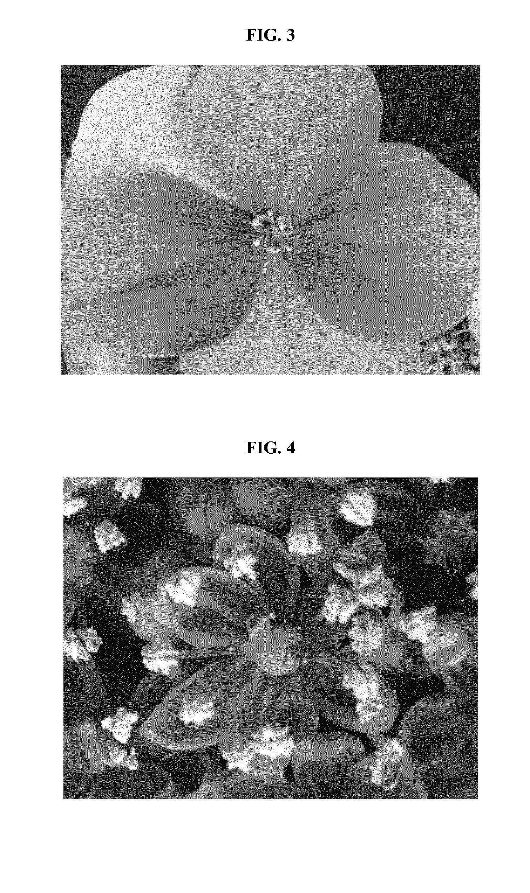

As used herein, a **tepaloid flower** has at least 4 tepaloids. For example, see Figures 1, 2, and 3. A non-tepaloid flower has no tepaloids and contains very small sepals, small petals, anthers and stamens. For instance, see Figure 4. These non-showy flowers are abundantly present in the centre of most lacecap inflorescences. The petals and anthers easily drop causing a lot of "dust" under potted Hydrangea plants and cut flowers with many non-tepaloid flowers, which is experienced as a very negative trait by the end user. For this reason cultivars with more tepaloids are highly desired.

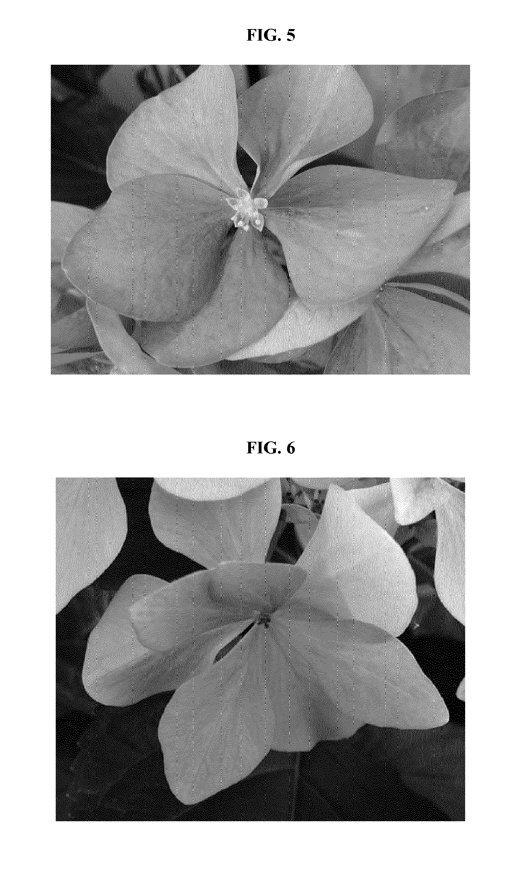

**Double flower** means that the flower has any number between 7 and 25 small to large tepaloids, instead of the usual 4-6. Flowers with less than 7 tepaloids are referred to as single. The more tepaloids a flower contains, the less petals and anthers are present. See, for example, Figures 5-7.

The degree of doubleness of a flower is defined by the number of tepaloids it contains. The more tepaloids per flower, the higher its degree of doubleness. For example, see Figures 3, 5, 6, 7, 10, 11. Tepaloid flowers may occur in any type of inflorescence. In lacecap inflorescences, the tepaloid flowers may occur only in the rim of the inflorescence as well as in the centre and the rim and, in this case, the tepaloids of the rim flowers usually are much bigger than the tepaloids of the central flowers. Also the degree of doubleness between rim and central flowers may differ, although this may be caused by the fact that the central flowers develop later than the rim flowers.

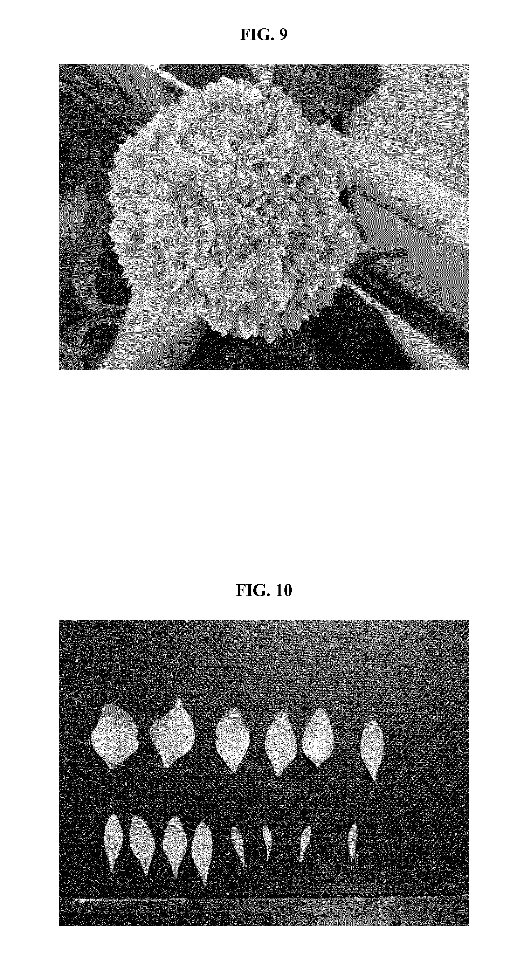

As used herein, a mophead inflorescence is defined by its half or fully spherical shape, containing many tepaloid flowers and, usually, also some either or not dried-up non-tepaloid flowers in the centre of the inflorescence. For example, see Figures 2, 9. Because these dried-up flowers usually can only be seen when the showy tepaloid flowers are taken away, the appearance of the mophead inflorescence is a showy, half to full sphere arrangement of tepaloid flowers. Mophead inflorescence thus means that at the fully flowering stage, the inflorescence looks like a half or full sphere and contains only flowers with large tepaloids.

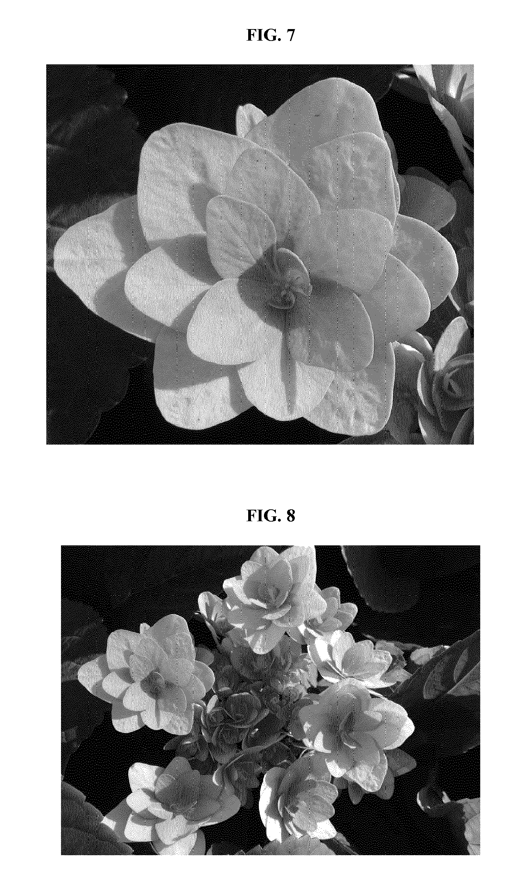

As used herein, a **lacecap inflorescence** is defined as a more or less flat, disk-like inflorescence with a rim of relatively few, showy, tepaloid flowers and a centre of relatively many, usually non-tepaloid flowers. For example, see Figures 1 and 8.

As used herein, a **hybrid** is defined as a plant in the progeny from a cross between 2 Hydrangea macrophylla plants, as well as a plant in the progeny from this cross with a single backcross or serial backcrosses to one of the parents or a similar parent, as well as a plant in the progeny from this cross with a cross with a plant in the progeny of the same cross or related crosses, which are all done to combine the double flower, mophead inflorescence and cold-period independent flowering trait which each other and/or with other desired traits.

### Breeding Program

The breeding program to combine the cold-period independent flowering, double flower, and mophead inflorescence traits present in various Hydrangea macrophylla selections and cultivars started in 2000. During the execution of this program the following parents were selected for specific traits: Hydrangea macrophylla (H. m.) "Semperflorens", H. m. "Frillibet", H. m. "Endless Summer", H. m. You&Me® cultivars, H. m. "Wedding Gown". H. m. "Corsage", H. m. "Komachi", H. m. "Early Blue" and "Hot Red" and other HBA-proprietary cultivars, H. m. White from Flor Andina, H. m. "Temari-Uta", H. m. "Kuro-Hime", H. m. "Mikawa-Chidori". Although some of these cultivars and selections are described as Hydrangea serrata, or Hydrangea macrophylla ssp. serrata, or Hydrangea macrophylla var. macrophylla, or Hydrangea macrophylla var. normalis, which demonstrates the present confusion in Hydrangea classification, for reasons of simplicity, herein they are all referred to as Hydrangea macrophylla.

Hydrangea macrophylla "Semperflorens", "Frillibet" and "Endless Summer" plants may include advantageous traits like cold-period independent flowering, mophead inflorescence, short time between potting and flower initiation, and spontaneous branching, but may also have traits that should be avoided in new cultivars like faint flower colour, large leaves, weak bending stems, and relatively small single flowers.

Hydrangea macrophylla White plants from Flor Andina, Colombia, may include advantageous traits like cold-period independent flowering, mophead inflorescence, short time between potting and flower initiation, but may also have traits that should be avoided in new cultivars like excessive growth, very long internodes, big leaves, weak bending stems, and relatively small flowers.

Plants from Hydrangea macrophylla You&Me® cultivars, such as "Emotion", "Eternity", "Forever", may include advantageous traits like double flower, mophead inflorescence, strongly reduced presence of anthers in the double flower lacecap cultivars, but may also have traits that should be avoided in new cultivars like big leaves, too strong growth, weak bending stem, and cold-period dependent flowering.

Plants from Hydrangea macrophylla cultivars "Wedding Dress", "Corsage", and "Komachi" exhibit the advantageous trait double flower, but may also have traits that should be avoided in new cultivars like too excessive growth, big leaves, cold-period dependent flowering, weak bending stems, faint colours, and predominantly lacecap inflorescences.

Plants from Hydrangea macrophylla cultivars "Early Blue", "Hot Red" and other HBA proprietary cultivars may exhibit advantageous traits like compact growth, strong stems, small leaves, mophead inflorescence, and strong flower colours, but may also have traits that should be avoided in new cultivars like cold-period dependent flowering, and single flowers.

Plants from Hydrangea macrophylla "Temari-Uta" may exhibit advantageous traits like double flower, and mophead inflorescence, but may also have traits that should be avoided in new cultivars like big leaves, too vigorous growth, weak stems, and cold-period dependent flowering.

### Plant Growth Conditions

In order to combine advantageous traits present in the various Hydrangea macrophylla selected parent plants, crosses between these parents were made. The parents were grown as potted plants in the greenhouse using conditions that are essentially described by J.L. van Leeuwen, F.R. van Noort, P.A.F. van Paassen, N.A. Straver (2003) Teelt van Hydrangea. Potplant en snijbloem. PPO-report 585, 109 pp.

When necessary, plants were forced into flowering using a cold period of 8 weeks at 5 °C. From the selected mother plants most flowers were removed before flower opening and from the few remaining flowers the anthers when present were removed upon flower opening. Pollen was collected from the selected father plant and transferred with a paint brush or tweezers to the style of flowers on the selected mother plant. These flowers were labelled with the cross identification number. Fruits were allowed to develop on the mother plants for 4-6 months. The seeds from the fruits were collected and cleaned.

Seeds were sown in November in trays containing essentially the same soil mixture as used for the potted parent plants. The trays were cultured in the greenhouse at similar conditions as the potted parent plants. Germination of the seeds generally was observed within 2-3weeks. Germinated seedlings were transplanted into new trays with the same potting soil, grown for 4 weeks, transferred to pots in January and grown in the greenhouse under long-day (18 h light per day) conditions. The greenhouse temperature was kept above 17 °C and additional lighting was given when needed to keep the light intensity above 3000 lux.

For selecting the seedlings for cold-period independent flowering, half of the potted seedlings were cultured for 6 months. Seedlings were selected for flowering in May and June. Flowering seedlings were marked as having the cold-period independent flowering trait and were selected for the following traits: double flower, mophead inflorescence, flower colour, stem rigidness and compact habitus.

From the other half of the potted seedlings one cutting per seedlings was taken in April, after rooting potted and pinched in May and transferred to the field in June. In October these potted plants were moved to the cold. In January the plants were moved to the greenhouse and grown into flowering that occurred in March-April. Also these seedlings were selected for the traits double flower, mophead inflorescence, flower colour, stem rigidness and compact habitus. This growing scheme for selection of the right combinations of traits is given in Figure 12.

In order to determine the stability of the traits double flower, mophead inflorescence and cold-period independent flowering, selected seedlings were propagated by cuttings in March to create a clone of 10 plants per seedling. The rooted cuttings were potted in April and grown essentially as described for the seedlings. In all cases were the seedling was selected for the combination of the traits mophead inflorescence and/or double flower with cold-period independent flowering, all plants of the clone made of this seedling expressed the same combination of traits giving evidence to the stable transmission of these trait combinations in vegetative propagation.

### Hydrangea Plants

Using this breeding and selection procedure for several successive years, the inheritance of some important traits in Hydrangea macrophylla plants was determined. All crosses between lacecap and mophead Hydrangea macrophylla plants resulted either in a progeny in which 100% of the plants had lacecap inflorescences or a progeny in which 50% of the plants had lacecap inflorescences and the other 50% had mophead inflorescences. Evidently, the trait lacecap inflorescence is dominant over the trait mophead inflorescence in Hydrangea macrophylla. A mophead inflorescence is only expressed in Hydrangea macrophylla plants that contain the recessive gene for this trait in homozygous form.

All crosses between Hydrangea macrophylla plants with the double flower trait and Hydrangea macrophylla plants with the single flower trait resulted either in a progeny in which 100% of the plants showed the single flower trait or a progeny in which 50% of the plants showed the double flower trait and the other 50% of the plants showed the single flower trait. Evidently, also the single flower trait in Hydrangea macrophylla plants is dominant over the double flower trait. The double flower trait in Hydrangea macrophylla plants is only expressed when the recessive gene for double flower is present in homozygous form.

The expression of the trait cold-period independent flowering in Hydrangea macrophylla plants was strongly influenced by the culture conditions of the plants. When the greenhouse temperature was well kept above 17 °C and the light intensity was well above 3000 lux for 18 hours per day, the expression of the cold-period independent flowering trait was optimal. Under these conditions the plants in a progeny of the cross between a Hydrangea macrophylla plant that expressed this trait and a Hydrangea macrophylla plant that did not express this trait expressed the cold-period independent flowering trait for 60-70%.

The following examples represent embodiments and are illustrative. These examples are non-limiting, and one of ordinary skill in the art would understand that many variations and modifications can be made while remaining within the spirit and scope of the present disclosure.

### EXAMPLE 1: Hydrangea having cold-independent flowering, mop-head inflorescence, and rigid stems

This example shows how the trait cold-period independent flowering from Hydrangea macrophylla "Frillibet" used as father was successfully combined with the traits mophead inflorescence and rigid stems in HBA-owned cultivars "Sweet Dreams" and "Pink Delight" in just two steps that took only two years in time.

Hydrangea macrophylla plant 09-0248-118 was selected on basis of the following combination of traits: cold-period independent flowering, solid mophead inflorescence, pink single flowers, small dark-green leaves and rigid stems. The plant was selected in the progeny from cross 09-0248, in which 100% of the plants carried a mophead inflorescence, between the HBA-owned Hydrangea macropylla cultivar "Pink Delight" (nr. 01-0215-010) as father, carrying the traits mophead inflorescence, small leaves and rigid stems, and plant 02-0147-029 as mother carrying the traits cold-period independent flowering and mophead inflorescence. This mother plant had been selected from a progeny of the earlier cross 02-0147, in which 50% of the plants carried a lacecap inflorescence, between the HBA-owned Hydrangea macrophylla cultivar "Sweet Dreams" (nr. 02-0004-000-13/01) as mother, carrying the traits lacecap inflorescence, but also one copy of the recessive gene for the trait mophead inflorescence, small leaves and rigid stems, and Hydrangea macropylla cultivar "Frillibet" as father (nr. 01-0036-000), carrying the traits mophead inflorescence and cold-period independent flowering.

### EXAMPLE 2: Hydrangea having cold-independent flowering, mophead inflorescence, and solid stems

This example demonstrates how the trait cold-independent flowering from Hydrangea macrophylla "Frillibet" used as father was successfully combined with the traits mophead inflorescence and solid stems in HBA-owned cultivars "Sweet Dreams" and "Royal Navy" in just two steps that took only two years.

Hydrangea macrophylla plant 10-0183-051 was selected on basis of the following combination of traits: cold-period independent flowering, rigid mophead inflorescence, pink single flowers, rigid stems, middle-sized dark-green leaves, and compact growth. The plant was selected in the progeny from cross 10-0183, in which 100% of the plants carried a mophead inflorescene, between the HBA-owned Hydrangea macrophylla cultivar "Royal Navy" (nr. 03-0134-055) as father, carrying the traits mophead inflorescence, rigid stems and small leaves, and Hydrangea macrophylla plant 02-0147-29 as mother, carrying the traits mophead inflorescence and cold-period independent flowering. This mother plant had been selected from the progeny of the earlier cross 02-0147, in which 50% of the plants carried a lace-cap inflorescence, between the HBA-owned Hydrangea macrophylla cultivar "Sweet Dreams" (nr. 02-0004-000-13/01) as mother, carrying the traits lacecap inflorescence, small leaves and rigid stems, and Hydrangea macropylla cultivar "Frillibet" as father (nr. 01-0036-000), carrying the traits mophead inflorescence and cold-period independent flowering.

### EXAMPLE 3: Recessive trait double flower can be combined with rigid stems, compact growth, and small leaves

This example shows how the recessive trait double flower from the Hydrangea macrophylla You&me® cultivars "Emotion" and "Forever" could be combined with the traits rigid stems, compact growth and small leaves from the HBA-owned Hydrangea macrophylla cultivar "Royal Red" and Hydrangea macrophylla cultivar "Moritzburg" in just 2 breeding steps which took 4 years.

Hydrangea macrophylla plant 12-0154-11 was selected on basis of the following combination of traits: double dark-pink flower, lacecap inflorescence, rigid stems, compact growth and small dark-green leaves.

The plant was selected in the progeny from cross 12-0154, in which 25% of the plants carried the trait double flower, between Hydrangea macrophylla plant 10-0170-098 as mother, carrying the traits single flower, short rigid stems, and small leaves, and Hydrangea macrophylla plant 10-0167-051 as father, carrying the traits single flower, short rigid stems, and small leaves. This mother plant had been selected in the progeny from the earlier cross 10-0170, in which all the plants carried the trait single flower, between Hydrangea macrophylla You&me® cultivar "Emotion" (nr. 08-0020-000) as mother, carrying the traits double flower and lacecap inflorescence, and the HBA-owned Hydrangea macrophylla cultivar "Royal Red" as father, carrying the traits rigid stems, compact growth, small leaves and strong flower colour. The father plant of cross 12-0154 (Hydrangea macrophylla nr. 10-0167-051) was selected in the progeny of cross 10-0167, in which all plants carried single flowers, between Hydrangea macrophylla You&me® cultivar "Forever" as mother, carrying the traits double flower and lacecap inflorescence, and Hydrangea macrophylla cultivar "Moritzburg" (nr. 00-0021-000) as father, carrying the traits mophead inflorescence, and single pink flower.

### EXAMPLE 4: Recessive trait double flower can be combined with rigid stems, compact growth, and small leaves

This example also shows how the recessive trait double flower from the Hydrangea macrophylla You&me® cultivars "Eternity" and "Forever" could be combined with the traits rigid stems, compact growth and small leaves from the HBA-owned Hydrangea macrophylla cultivar "Duro" and Hydrangea macrophylla cultivar "Moritzburg" in just 2 breeding steps which took 4 years. Example 3 and 4 show, that the recessive trait of double flower can be introduced via the mother and the father into a progeny that completely carries single flowers, but in which 50% of the plants carry the gene for the double flower trait. Crossing selected plants within these progenies results in a progeny, in which 25% of the plants again show the double flower trait.

Hydrangea macrophylla plant 12-0133-256 was selected on basis of the following combination of traits: dark-pink double flower, rigid mophead inflorescence, rigid stem, small dark-green leaves and compact growth.

The plant was selected in the progeny from cross 12-0133, in which 25% of the plants carried the double flower trait, between Hydrangea macrophylla plant 10-0169-002 as mother, carrying the traits single flower, short rigid stems, small leaves, strong colour, and Hydrangea macrophylla plant 10-0167-091 as father, carrying the traits single flower, short rigid stems, and small leaves. This mother plant had been selected in the progeny from the earlier cross 10-0169, in which none of the plants carried the double flower trait, between Hydrangea macrophylla You&me® cultivar "Eternity" as mother, carrying the traits double flower and lacecap inflorescence, and the HBA-owned Hydrangea macrophylla cultivar "Duro" as father, carrying the traits single flower, mophead inflorescence, rigid stem and compact growth. The father of cross 12-0133, Hydrangea macrophylla plant 10-0167-091, was selected from the progeny of the earlier cross 10-0167, in which none of the plants carried the double flower trait, between Hydrangea macrophylla You&me® cultivar "Forever" as mother, carrying the traits double flower and lacecap inflorescence, and Hydrangea macrophylla cultivar "Moritzburg (nr. 00-0021-000) as father, carrying the traits mophead inflorescence, and single pink flower.

### EXAMPLE 5: Recessive traits double flower and mophead can be combined with cold-period independent flowering, rigid stems, compact growth, and small leaves

This example shows that, despite the two recessive traits double flower and mophead inflorescence, it is very well possible to combine these traits with the traits cold-period independent flowering, rigid stems, compact growth and small leaves using only 3 parents, 2 breeding cycles, and 3 years' time.

Hydrangea macrophylla plant 12-0157-006 was selected on basis of the following combinations of traits: cold-period independent flowering, double flower, mophead inflorescence, rigid stem, small leaves, and compact growth. The plant was selected in the progeny of cross 12-0157, in which 25% of the plants carried the trait double flower, and 60% of the plants carried the trait cold-period independent flowering. Cross 12-0157 was made using Hydrangea macrophylla plant 10-0171-014 as mother, carrying the traits cold-period independent flowering, weak bending stems and one copy of the recessive gene for double flower, and Hydrangea macrophylla plant 10-0170-047 as father, carrying the traits strong rigid stem, small leaves, cold-period dependent flowering, and one copy of the recessive gene for double flower. The mother of cross 12-0157 was selected in the progeny of cross 10-0171, in which all plants carried the trait single flower and about 60% of the plants carried the trait cold-period independent flowering, from a cross between Hydrangea macrophylla You&me® cultivar "Emotion" (nr. 08-0020-000) as mother, carrying the traits double flower and lacecap inflorescence, and Hydrangea macrophylla cultivar "Colombia White" (nr. 10-0001-000) as father, carrying the traits cold-period independent flowering, and mophead inflorescence. The father of cross 12-0157 was selected in the progeny of cross 10-0170, in which all plants carried the trait single flower and many had rigid stems, small leaves and mophead inflorescences. Cross 10-170 was made using Hydrangea macrophylla You&me® cultivar "Emotion" as mother and the HBA-owned Hydrangea macrophylla cultivar "Royal Red" as father, carrying the traits single red flower, mophead inflorescence, rigid stem, small leaves, and compact growth.

### EXAMPLE 6: Recessive traits double flower and mophead can be combined with cold-period independent flowering, rigid stems, compact growth, and small leaves in a short period of time.

This example shows that, despite the presence of two recessive traits double flower and mophead inflorescence, it is very well possible to combine these traits with the traits cold-period independent flowering, rigid stems, compact growth and small leaves using only 4 parents, 2 breeding cycles and 3 years time.

Hydrangea macrophylla plant 12-160-002 was selected on basis of the following combination of traits: cold-period independent flowering, double flower, mophead inflorescence, and rigid stem.

The plant was selected in the progeny from cross 12-0160, in which 25% of the plants carried the trait double flower, and 60% of the plants carried the trait cold-period independent flowering. Cross 12-0160 was made using Hydrangea macrophylla plant 10-0171-021 as mother, carrying the traits cold-period independent flowering and single flower, and Hydrangea macrophylla plant 10-0195-047 as father, carrying the traits single flower plus one copy of the recessive gene for double flower, strong rigid stems and small leaves. The mother of cross 12-0160 was selected in the progeny of cross 10-0171, in which all plants carried the trait single flower and about 60% of the plants carried the trait cold-independent flowering, from a cross between Hydrangea macrophylla You&me® cultivar "Emotion" (nr. 08-0020-000) as mother, carrying the traits double flower and lacecap inflorescence, and Hydrangea macrophylla cultivar "Colombia White" (nr. 10-0001-000) as father, carrying the traits cold-period independent flowering, and mophead inflorescence. The father of cross 12-0160 was selected in the progeny of cross 10-0195, in which all plants carried the trait single flower plus one copy of the recessive gene for double flower, strong rigid stems and small leaves. Cross 10-0195 was made using Hydrangea macrophylla cultivar "Dance Party" (nr. 09-0003-000) as mother and the HBA-owned Hydrangea macrophylla cultivar "Royal Navy" as father, carrying the traits mophead inflorescence, rigid stems and small leaves.

### EXAMPLE 7: Hydrangea having cold-period independent flowering, mophead inflorescence and double flower can be produced within two breeding cycles and in two years' time

Example 7 clearly shows, that it is possible to combine the traits cold-period independent flowering, mophead inflorescence and double flower in two breeding cycles taking two years' time.

Hydrangea macrophylla plant 12-0162-031 was selected on basis of the following combination of traits: cold-period independent flowering, double flower, and mophead inflorescence. The plant also has a weak bending stem and big leaves. The plant was selected in the progeny from cross 12-0162, in which 100% of the plants carried the trait mophead inflorescence, 50% carried the trait double flower and ca 60% of the plants carried the trait cold-period independent flowering. Cross 12-0162 was made between Hydrangea macrophylla plant nr. 10-0171-023 as mother, carrying the traits mophead inflorescence, single flower and cold-period independent flowering, and Hydrangea macrophylla plant nr. 10-0171-021 as father, carrying the traits mophead inflorescence, single flower and cold-period independent flowering. Both parents also carried one gene for the recessive trait double flower. Both parents of cross 12-0162 where selected in the progeny of cross 10-0171, in which all plants carried the trait single flower and one copy of the recessive gene for double flower, 50% had a mophead inflorescence and about 60% of the plants carried the trait cold-period independent flowering, made between Hydrangea macrophylla You&me® cultivar "Emotion" (nr. 08-0020-000) as mother, carrying the traits double flower and lacecap inflorescence, and Hydrangea macrophylla cultivar "Colombia White" (nr. 10-0001-000) as father, carrying the traits cold-period independent flowering, and mophead inflorescence.

## Claims

1. A Hydrangea plant having one or more traits selected from the group consisting of cold-period independent flowering, mophead inflorescence, and double flower.

2. The Hydrangea plant of claim 1, wherein said plant has the traits cold-period independent flowering, and double flower.

3. The Hydrangea plant of claim 1, wherein said plant has the traits cold-period independent flowering, and mophead inflorescence.

4. The Hydrangea plant of claim 1, wherein said plant has the traits cold-period independent flowering, mophead inflorescence, and double flower

5. A method for breeding a Hydrangea hybrid plant having the traits cold-period independent flowering, mophead inflorescence, and double flower comprising:
a. crossing a cold-period independent flowering Hydrangea macrophylla plant, either as male or female parent, with any Hydrangea macrophylla plant;
b. selecting a plant from the progeny of such cross that flowers within 8 months of potting the rooted cuttings due to the presence of the cold-period independent flowering trait and exhibits the traits mophead inflorescence and double flower.

6. A method for breeding a Hydrangea hybrid plant having the traits cold-period independent flowering, and double flower, comprising:
a. crossing a cold-period independent flowering Hydrangea macrophylla plant, either as male or female parent, with any Hydrangea macrophylla plant;
b. selecting a plant from the progeny of such cross that flowers within 8 months of potting the rooted cutting due to the presence of the cold-period independent flowering trait and exhibits the trait double flower.
